Europäisches Patentamt

European Patent Office

Office européen des brevets

(19)

(11) Numéro de publication : **0 344 043 B1**

(12)                    **FASCICULE DE BREVET EUROPEEN**

(45) Date de publication du fascicule du brevet :
**18.03.92 Bulletin 92/12**

(51) Int. Cl.$^5$ : **C07C 47/21, C07C 45/51**

(21) Numéro de dépôt : **89401334.1**

(22) Date de dépôt : **12.05.89**

(54) **Procédé de préparation du citral.**

(30) Priorité : **16.05.88 FR 8806524**

(43) Date de publication de la demande :
**29.11.89 Bulletin 89/48**

(45) Mention de la délivrance du brevet :
**18.03.92 Bulletin 92/12**

(84) Etats contractants désignés :
**AT BE CH DE ES FR GB GR IT LI LU NL SE**

(56) Documents cités :
**EP-A- 0 021 074
DE-A- 2 411 530
DE-A- 2 423 409
PATENT ABSTRACTS OF JAPAN, vol. 10, no.
172 (C-354)[2228], 18 juin 1986, page 81 C 354;
& JP-A-61 22 038
SYNTHESIS, no. 2, février 1983, page 165,
résumé no. 6553; B.H. LIPSHUTZ et al.:
"Cleavage of acetals"**

(73) Titulaire : **RHONE-POULENC SANTE
20, avenue Raymond Aron
F-92160 Antony (FR)**

(72) Inventeur : **Chabardes, Pierre
24 rue Jeanne d'Arc
F-69110 Sainte Foy les Lyon (FR)**
Inventeur : **Chazal, Jacques
78 avenue Jean Jaurés
F-69190 Saint-Fons (FR)**

(74) Mandataire : **Le Pennec, Magali et al
RHONE-POULENC SANTE, Service Brevets,
20 Avenue Raymond Aron
F-92165 Antony Cédex (FR)**

**Description**

La présente invention concerne un procédé de préparation du citral à partir du prénal et du prénol.

D'après le brevet français FR 74 40959 (2 246 529), il est connu de préparer le citral par craquage de l'acétal diprénylique du prénal qui est lui-même obtenu par condensation de deux moles de prénol sur une mole de prénal. Selon le brevet français FR 74 40959 (2 246 529) la condensation du prénol sur le prénal est effectuée en présence d'un agent de condensation qui, de préférence, est un sel d'ammonium tel que le nitrate d'ammonium, et d'un agent de déshydratation qui peut être du sulfate de calcium ou un tamis moléculaire, et la pyrolyse de l'acétal diprénylique ainsi obtenu est effectuée par chauffage à une température comprise entre 120 et 230°C éventuellement en présence d'un catalyseur acide tel que les acides p.toluènesulfonique ou o.nitrobenzoïque ou le sulfate acide de sodium.

Il a maintenant été trouvé, et c'est ce qui fait l'objet de la présente invention, que la condensation et la pyrolyse peuvent être effectuées en utilisant comme catalyseur un halogénure de lithium et, plus particulièrement, le chlorure de lithium.

Généralement, l'acétalisation est effectuée en utilisant au moins deux moles de prénol par mole de prénal et en opérant dans un solvant organique inerte capable d'entraîner l'eau formée par distillation azéotropique tel qu'un hydrocarbure aromatique comme le toluène, à une température comprise entre 70 et 100°C. Il peut être avantageux d'opérer sous une pression réduite qui est généralement comprise entre 20 et 100 mm de mercure (2,7 à 13,3 kPa) selon la nature du solvant utilisé.

Le craquage de l'acétal diprénylique du prénal est généralement effectué par chauffage à une température comprise entre 120 et 150°C en présence d'un halogénure de lithium, de préférence le chlorure de lithium en opérant dans un solvant organique convenable tel que le dichloro-1,2 benzène. Il peut être avantageux d'opérer sous une pression réduite généralement voisine de 100 mm de mercure (13,3 kPa).

Selon le procédé de la présente invention, il n'est pas nécessaire d'isoler l'acétal diprénylique du prénal avant de procéder au craquage. Dans ce cas, il n'est pas nécessaire d'ajouter un halogénure de lithium pour effectuer le craquage, le catalyseur étant l'halogénure de lithium contenu dans le produit brut issu de la phase d'acétalisation.

Pour la mise en oeuvre des étapes d'acétalisation et de craquage, il peut être particulièrement avantageux d'opérer en présence d'un inhibiteur de polymérisation tel que l'hydroquinone.

Généralement, pour la mise en oeuvre du procédé selon l'invention, on utilise de 0,01 à 0,1 mole d'halogénure de lithium par mole de prénal ou d'acétal diprénylique du prénal.

Le procédé selon l'invention permet d'obtenir sélectivement le citral avec des rendements généralement supérieurs à 60 % par rapport au prénal transformé, le taux de transformation du prénal étant généralement supérieur à 75 %.

L'exemple suivant, donné à titre non limitatif, illustre la présente invention.

EXEMPLE

Dans un ballon muni d'une colonne à distiller, on introduit 35,1 g de prénal dont la pureté est de 97,2 % (405,58 mmoles) et 87 g de prénol pur (1010,22 mmoles), 11,5 cm3 de toluène et 0,1 g d'hydroquinone. On ajoute 0,34 g de chlorure de lithium (8,02 mmoles) puis chauffe pendant 2 heures 30 minutes sous pression réduite (80 mm de mercure ; 10,7 kPa) à une température comprise entre 75 et 79°C en récupérant l'eau entraînée par azéotropie (3 cm3). On ajoute à nouveau 0,34 g de chlorure de lithium puis chauffe encore pendant 2 heures sous pression réduite (80 mm de mercure ; 10,7 kPa) à une température comprise entre 82 et 86°C en récupérant l'eau entraînée par azéotropie (5,2 cm3 au total). Après élimination du toluène par distillation, le ballon contient 117,68 g d'une huile jaune pâle constituée essentiellement d'acétal diprénylique du prénal.

L'analyse du mélange réactionnel montre que le taux de transformation du prénal est de 73 % et que le rendement en acétal diprénylique du prénal est de 93 % par rapport au prénal transformé.

A 116,08 g de l'acétal diprénylique du prénal brut obtenu précédemment titrant 55,8 % soit 271,74 mmoles on ajoute 10 g de dichloro-1,2 benzène.

Après avoir éliminé l'excès de prénol et le prénal n'ayant pas réagi par distillation sous pression réduite (15 mm de mercure ; 2 kPa), le mélange est chauffé sous pression réduite (90 mm de mercure ; 12 kPa) pendant 3 heures 30 minutes à une température comprise entre 135 et 142°C. On recueille 80 cm3 (70,9 g) de distillat. Le résidu dans le ballon (54,2 g) se présente sous forme d'une huile jaune orangé constituée essentiellement de citral.

L'analyse du mélange réactionnel montre que le taux de transformation de l'acétal diprénylique du prénal est de 70 % et que le rendement en citral est de 96 % par rapport à l'acétal transformé.

**Revendications**

1. Procédé de préparation du citral par condensation d'au moins deux moles de prénol par mole de prénal suivie du craquage de l'acétal diprénylique du prénal caractérisé en ce que l'on utilise comme catalyseur d'acétalisation et de craquage un halogénure de lithium.

2. Procédé selon la revendication 1 caractérisé en ce que l'halogénure de lithium est le chlorure de lithium.

3. Procédé selon l'une des revendications 1 ou 2 caractérisé en ce que l'on utilise 0,01 à 0,1 mole de catalyseur par mole de prénal mis en oeuvre.

4. Procédé selon la revendication 1 caractérisé en ce que l'acétalisation en présence d'un halogénure de lithium est effectuée à une température comprise entre 70 et 100°C dans un solvant organique inerte permettant d'éliminer l'eau formée par distillation azéotropique, et en opérant éventuellement sous pression réduite.

5. Procédé selon la revendication 1 caractérisé en ce que le craquage en présence d'un halogénure de lithium est effectué à une température comprise entre 120 et 150°C en opérant éventuellement sous pression réduite.

6. Procédé selon la revendication 5 caractérisé en ce que l'halogénure de lithium utilisé dans la phase de craquage est celui qui est présent dans le produit brut obtenu dans la phase d'acétalisation.

**Patentansprüche**

1. Verfahren zur Herstellung von Citral durch Kondensation von zumindest zwei mol prenol pro mol prenal und anschließendes Kracken des Prenal-diprenylacctals, dadurch gekennzeichnet, daß man als Katalysator für die Acetalisierung und das Kracker ein Lithiumhalogenid verwendet.

2. Verfahren nach Anspruch 1, dadurch gekennzeichnet, daß das Lithiumhalogenid Lithiumchlorid ist.

3. Verfahren nach einem der Ansprüche 1 oder 2, dadurch gekennzeichnet, daß man 0,01 bis 0,1 mol Katalysator pro mol eingesetztes Prenal verwendet.

4. Verfahren nach Anspruch 1, dadurch gekennzeichnet, daß die Acetalisierung in Gegenwart eines Lithiumhalogenids bei einer Temperatur zwischen 70 und 100°C in einem inerten organischen lösungsmittel ausgeführt wird, das es ermöglicht, das gebildete Wasser durch azeotrope Destillation zu entfernen, und indem man gegebenenfalls unter vermindertem Druck arbeitet.

5. Verfahren nach Anspruch 1, dadurch gekennzeichnet, daß das Kracken in Gegenwart eines Lithiumhaloyenids bei einer Temperatur zwischen 120 und 150°C ausgeführt wird, indem man gegebenenfalls unter vermindertem Druck arbeitet.

6. Verfahren nach Anspruch 5, dadurch gekennzeichnet, daß das in der Krackphase verwendete Lithiumhalogenid jenes ist, das in dem in der Acetalisierungsphase erhaltenen Rohprodukt vorhanden ist.

**Claims**

1. Process for the preparation of citral by condensation of at least two moles of prenol per mole of prenal followed by cracking of the diprenyl acetal of prenal, characterised in that a lithium halide is employed as an acetalization and cracking catalyst.

2. Process according to Claim 1, characterised in that the lithium halide is lithium chloride.

3. Process according to either of Claims 1 and 2, characterised in that 0.01 to 0.1 mole of catalyst is employed per mole of prenal introduced.

4. Process according to Claim 1, characterised in that the acetalization in the presence of a lithium halide is carried out at a temperature of between 70 and 100°C in an inert organic solvent enabling the water formed to be removed by azeotropic distillation, and with the operation being carried out under reduced pressure if desired.

5. Process according to Claim 1, characterised in that the cracking in the presence of a lithium halide is carried out at a temperature of between 120 and 150°C, the operation being carried out under reduced pressure if desired.

6. Process according to Claim 5, characterised in that the lithium halide employed in the cracking stage is that which is present in the crude product obtained in the acetalization stage.